# EUROPEAN PATENT SPECIFICATION

(11) **EP 3 956 907 B1**
(45) Date of publication and mention of the grant of the patent: **20.08.2025**
(21) Application number: 20718305.4
(22) Date of filing: 09.04.2020
(51) Int. Cl.: G16H 50/20, G16H 50/30

(54) **DIGITAL BIOMARKER**
DIGITALER BIOMARKER
BIOMARQUEUR NUMÉRIQUE

(30) Priority: 15.04.2019 EP 19169249
(43) Date of publication of application: 23.02.2022
(73) Proprietor: F. Hoffmann-La Roche AG, 4070 Basel (CH)
(72) Inventor: MEIER, Irene, B., 4051 Basel (CH); GOSSENS, Christian, 4070 Basel (CH); LINDEMANN, Michael, 4051 Basel (CH); KILCHENMANN, Timothy, 4070 Basel (CH); POINTEAU, Gregoire, Henry, Simon, 4070 Basel (CH); IBANEZ ALONSO, Luis, Rafael, 4070 Basel (CH); TAYLOR, Kirsten, 4070 Basel (CH)
(74) Representative: Müller-Afraz, Simona
(86) International application number: PCT/EP2020/060185
(87) International publication number: WO 2020/212251

(56) References cited:
- LAMPROS C. KOURTIS ET AL: "Digital biomarkers for Alzheimer's disease: the mobile/wearable devices opportunity", NPJ DIGITAL MEDICINE, vol. 2, no. 1, 21 February 2019 (2019-02-21), pages 1 - 9, XP055626285, DOI: 10.1038/s41746-019-0084-2
- SERGEY O. BACHURIN ET AL: "Drugs in Clinical Trials for Alzheimer's Disease: The Major Trends : DRUGS IN CLINICAL TRIALS FOR ALZHEIMER'S DISEASE", MEDICINAL RESEARCH REVIEWS, vol. 37, no. 5, 1 September 2017 (2017-09-01), US, pages 1186 - 1225, XP055626589, ISSN: 0198-6325, DOI: 10.1002/med.21434

## Description

### FIELD

Present invention relates to a medical device for improved patient testing and patient analysis. More specifically, aspects described herein provide diagnostic devices, systems and methods for assessing symptom severity and progression of Alzheimer's disease in a patient by active testing and/or passive monitoring of the patient.

### BACKGROUND

Alzheimer's disease (AD) is a neurodegenerative disorder of the central nervous system and the leading cause of a progressive dementia in the elderly population. Its clinical symptoms are impairment of memory, cognition, temporal and local orientation, judgment and reasoning but also severe emotional disturbances. There are currently no treatments available which can prevent the disease or its progression or stably reverse its clinical symptoms. AD has become a major health problem in all societies with high life expectancies and also a significant economic burden for their health systems.

There are several standardized methods and tests for measuring the symptom severity and progression in patients diagnosed with Alzheimer's disease. Each of the tests involves a doctor measuring the subject's abilities to perform various mental and physical functions in different ways. These standardized tests can provide an assessment of the various symptoms associated with the patient's cognitive, behavioral, motor functions and capabilities and can help track changes in these symptoms over time. Assessing symptom severity and progression using standardized methods and tests can, therefore, help guide treatment and therapy options.

Semantic memory is impaired early in Alzheimer's disease (AD) and semantic dementia, and traditionally assessed through the Boston Naming Test (Kaplan 1983), where subjects are asked to select or differentiate presented images with increasing difficulty level.

McRae *et al.* lists semantic feature production norms for a large set of living and nonliving things (McRae et al. Behavioral Research Methods, Instruments, and Computers. 2005;37:547-559).

LAMPROS C. KOURTIS ET AL: "Digital biomarkers for Alzheimer's disease: the mobile/wearable devices opportunity",NPJ DIGITAL MEDICINE, vol. 2, no. 1, 21 February 2019, mention a growing interest in the identification of readily accessible digital biomarkers, which enable early diagnose of neurodegenerative diseases.

Currently, assessing the severity and progression of symptoms in a patient diagnosed with Alzheimer's disease involves in-clinic monitoring and testing of the patient every 6 to 12 months. While monitoring and testing a patient more frequently is ideal, increasing the frequency of in-clinic monitoring and testing can be costly and inconvenient to the patient.

### BRIEF SUMMARY

The following presents a simplified summary of various aspects described herein. This summary is not an extensive overview, and is not intended to identify key or critical elements or to delineate the scope of the claims. The following summary merely presents some concepts in a simplified form as an introductory prelude to the more detailed description provided below. Aspects described herein describe specialized medical devices for assessing the severity and progression of symptoms for a patient diagnosed with Alzheimer's disease. Testing and monitoring may be done remotely and outside of a clinic environment, thereby providing lower cost, increased frequency, and simplified ease and convenience to the patient, resulting in improved detection of symptom progression, which in turn results in better treatment.

According to one aspect, the disclosure relates to a diagnostic device for assessing one or more symptoms of Alzheimer's disease in a subject. The device includes at least one processor, one or more sensors associated with the device, and memory storing computer-readable instructions that, when executed by the at least one processor, cause the device to receive a plurality of first sensor data via the one or more sensors associated with the device, extract, from the received first sensor data, a first plurality of features associated with the one or more symptoms of Alzheimer's disease in the subject, and determine a first assessment of the one or more symptoms of Alzheimer's disease based on the extracted first plurality of features.

### BRIEF DESCRIPTION OF THE DRAWINGS

A more complete understanding of aspects described herein and the advantages thereof may be acquired by referring to the following description in consideration of the accompanying drawings, in which like reference numbers indicate like features, and wherein:
FIG. 1 is a diagram of an example environment in which a diagnostic device for assessing one or more symptoms of Alzheimer's disease in a subject is provided according to an example embodiment.
FIG. 2 is a flow diagram of a method for assessing one or more symptoms of Alzheimer's disease in a subject based on passive monitoring of the subject according to an example embodiment.
FIG. 3 is a flow diagram of a method for assessing one or more symptoms of Alzheimer's disease in a subject based on active testing of the subject according to an example embodiment.
FIG. 4 illustrates one example of a network architecture and data processing device that may be used to implement one or more illustrative aspects described herein.
FIGS 5-7 depict example screenshots each illustrating an example diagnostic application according to one or more illustrative aspects described herein.

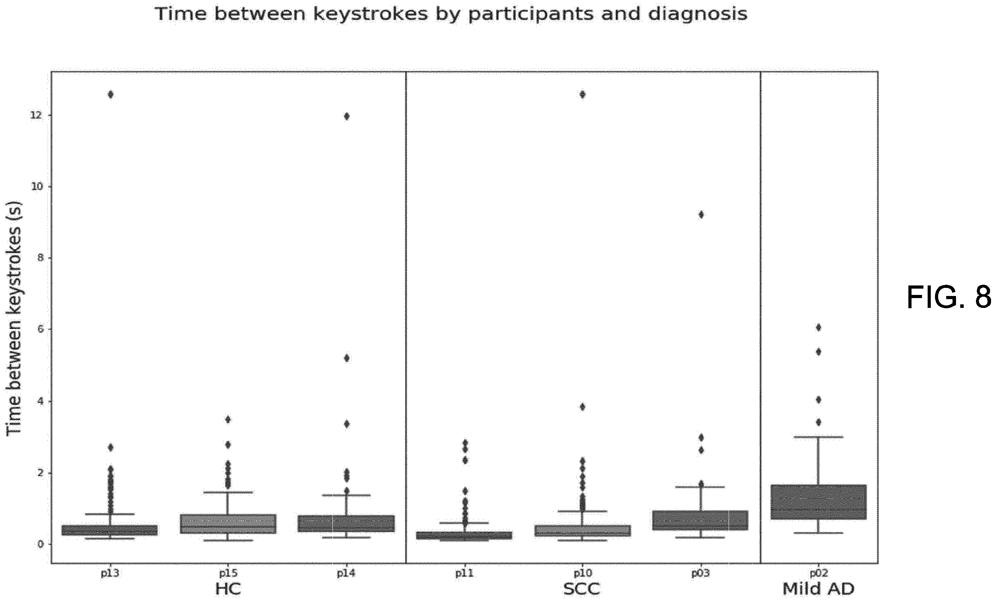

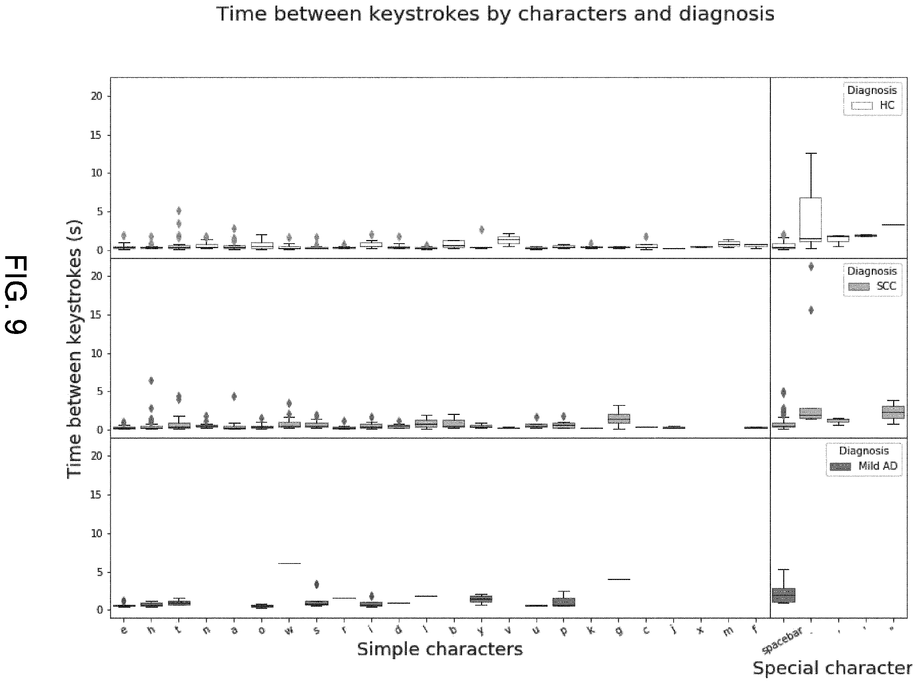

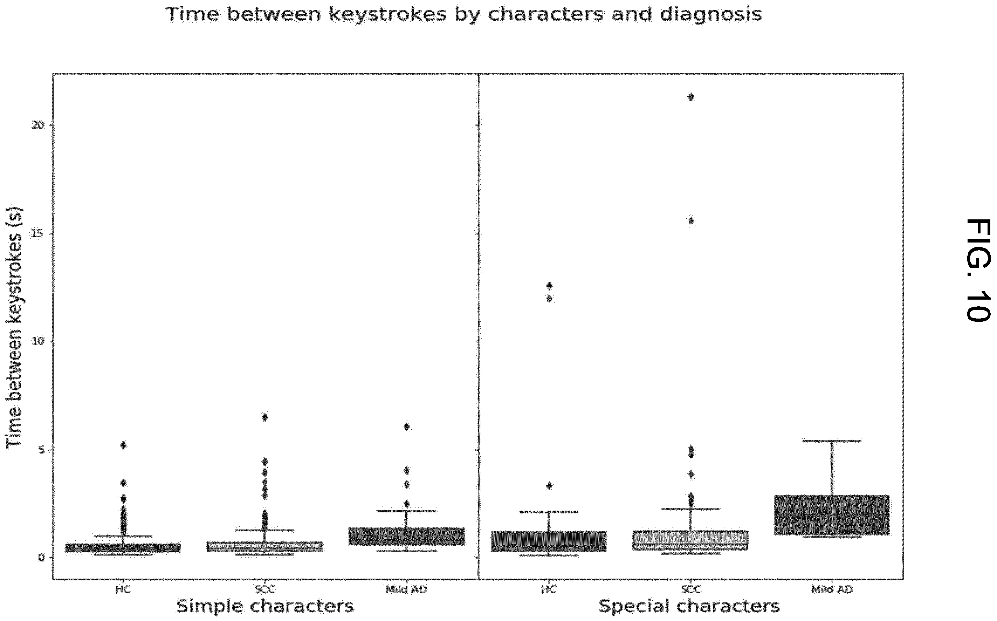

FIGS 8-10 are plots illustrating the time between keystrokes in a Fairytale test according to example 1.

### DETAILED DESCRIPTION

In the following description of various aspects, reference is made to the accompanying drawings, which form a part hereof, and in which is shown by way of illustration various embodiments in which aspects described herein may be practiced. It is to be understood that other aspects and/or embodiments may be utilized and structural and functional modifications may be made without departing from the described aspects and embodiments. Aspects described herein are capable of other embodiments and of being practiced or being carried out in various ways. Also, it is to be understood that the phraseology and terminology used herein are for the purpose of description and should not be regarded as limiting. Rather, the phrases and terms used herein are to be given their broadest interpretation and meaning. The use of "including" and "comprising" and variations thereof is meant to encompass the items listed thereafter as well as additional items.

The use of the terms "mounted," "connected," "coupled," "positioned," "engaged" and similar terms, is meant to include both direct and indirect mounting, connecting, coupling, positioning and engaging.

Systems, methods and devices described herein provide a diagnostic for assessing one or more symptoms of Alzheimer's disease in a patient. In some embodiments, the diagnostic may be provided to the patient as a software application installed on a mobile device.

In some embodiments, systems, methods and devices described herein provide a diagnostic for assessing one or more symptoms of Alzheimer's disease in a patient based on passive monitoring of the patient. In some embodiments, the diagnostic obtains or receives sensor data from one or more sensors associated with the mobile device as the patient performs activities of daily life. In some embodiments, the sensors may be within the mobile device like a smartphone or wearable sensors like a smartwatch. In some embodiments, the sensor features associated with the symptoms of Alzheimer's disease are extracted from the received or obtained sensor data. In some embodiments, the assessment of the symptom severity and progression of Alzheimer's disease in the patient is determined based on the extracted sensor features.

In some embodiments, systems, methods and devices according to the present disclosure provide a diagnostic for assessing one or more symptoms of Alzheimer's disease in a patient based on active testing of the patient. In some embodiments, the diagnostic prompts the patient to perform diagnostic tasks. In some embodiments, the diagnostic tasks are anchored in or modelled after established methods and standardized tests. In some embodiments, in response to the patient performing the diagnostic task, the diagnostic obtains or receives sensor data via one or more sensors. In some embodiments, the sensors may be within a mobile device or wearable sensors worn by the patient. In some embodiments, sensor features associated with the symptoms of Alzheimer's disease are extracted from the received or obtained sensor data. In some embodiments, the assessment of the symptom severity and progression of Alzheimer's disease in the patient is determined based on the extracted features of the sensor data.

Assessments of symptom severity and progression of Alzheimer's disease using diagnostics according to the present disclosure correlate sufficiently with the assessments based on clinical results and may thus replace clinical patient monitoring and testing. Example diagnostics according to the present disclosure may be used in an out of clinic environment, and therefore have advantages in cost, ease of patient monitoring and convenience to the patient. This facilitates frequent patient monitoring and testing, resulting in a better understanding of the disease stage and provides insights about the disease that are useful to both the clinical and research community. An example diagnostic according to the present disclosure can provide earlier detection of even small changes in symptoms of Alzheimer's disease in a patient and can therefore be used for better disease management including individualized therapy. Such intra-individual performance variability gives a hint at preclinical stages of the disease, before a patient exhibits any symptoms.

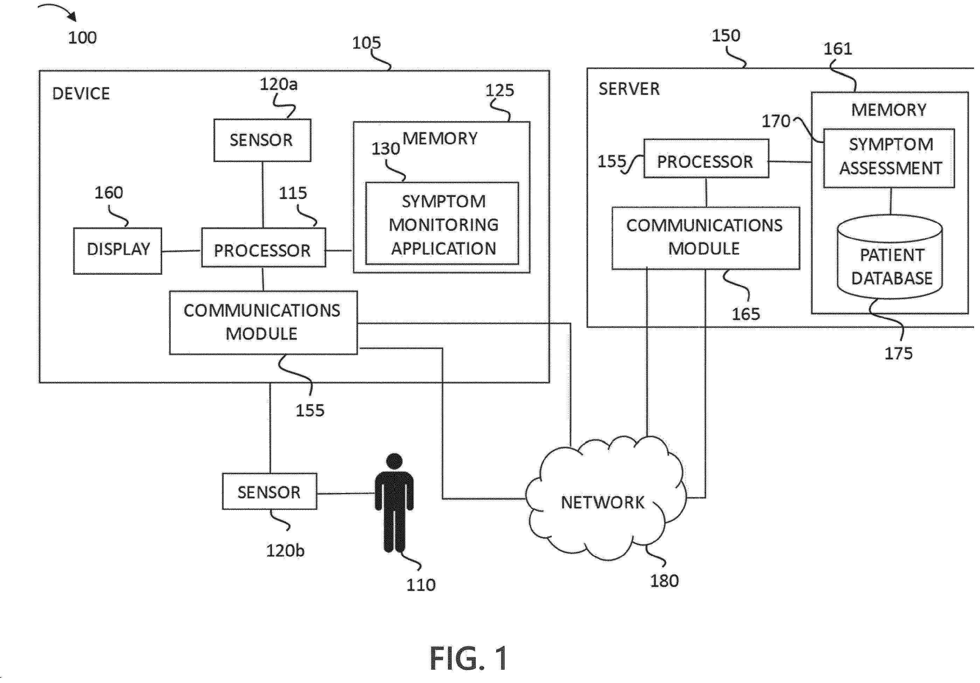

FIG. 1 is a diagram of an example environment 100 in which a diagnostic device 105 for assessing one or more symptoms of Alzheimer's disease in a subject 110 is provided. In some embodiments, the device 105 may be a smartphone, a smartwatch or other mobile computing device. The device 105 includes a display screen 160. In some embodiments, the display screen 160 may be a touchscreen. The device 105 includes at least one processor 115 and a memory 125 storing computer-instructions for a symptom monitoring application 130 that, when executed by the at least one processor 115, cause the device 105 to assess the one or more symptoms of Alzheimer's disease in the subject 110 based on passive monitoring of the subject 110. The device 105 receives a plurality of sensor data via one or more sensors associated with the device 105. In some embodiments, the one or more sensors associated with the device is at least one of a sensor disposed within the device or a sensor worn by the subject and configured to communicate with the device. In FIG. 1, the sensors associated with the device 105 include a first sensor 120a that is disposed within the device 105 and a second sensor 120b that is worn by the subject 110. The device 105 receives a plurality of first sensor data via the first sensor 120a and a plurality of second sensor data via the second sensor 120b as the subject 110 performs activities of daily life.

The device 105 extracts, from the received first sensor data and second sensor data, features associated with one or more symptoms of Alzheimer's disease in the subject 110. In some embodiments, the symptoms of Alzheimer's disease in the subject 110 may include a symptom indicative of a cognitive function of the subject 110, a symptom indicative of a motor function of the subject 110, or a symptom indicative of a functional capacity of the subject 110. In some embodiments, the one or more symptoms of Alzheimer's disease in the subject 110 are indicative of at least one of visual attention, motor speed, cognitive processing speed, visuo-motor coordination or fine motor impairment.

In some embodiments, the first sensor 120a or second sensor 120b (or another sensor altogether) associated with the device 105 may include or interface with a satellite-based radio navigation system, such as may be used with the Global Positioning System (GPS), Galileo, GLONASS, and/or similar systems (collectively referred to herein as GPS), and the plurality of first sensor data received from the first sensor 120b may include location data associated with the device 105. In some embodiments, the device 105 extracts location data, from the received first sensor data and second sensor data, associated with one or more symptoms of Alzheimer's disease in the subject 110. In some embodiments, an assessment of motor function of the subject 110 may be based at least in part on the extracted location data (e.g., patient mobility may be assessed based in part on GPS location data). In some embodiments, the sensors 120 associated with the device 105 may include sensors associated with Bluetooth and WiFi functionality and the sensor data may include information associated with the Bluetooth and WiFi signals received by the sensors 120. In some embodiments, the device 105 extracts data corresponding to the density of Bluetooth and WiFi signals received or transmitted by the device 105 or sensors, from the received first sensor data and second sensor data. In some embodiments, an assessment of behavioral function or an assessment of the functional capacity of the subject 110 may be based on the extracted Bluetooth and WiFi signal data (e.g., an assessment of patient sociability may be based in part on the density of Bluetooth and WiFi signals picked up).

The device 105 determines an assessment of the one or more symptoms of Alzheimer's disease in the subject 110 based on the extracted features of the received first and second sensor data. In some embodiments, the device 105 send the extracted features over a network 180 to a server 150. The server 150 includes at least one processor 155 and a memory 161 storing computer-instructions for a symptom assessment application 170 that, when executed by the server processor 155, cause the processor 155 to determine an assessment of the one or more symptoms of Alzheimer's disease in the subject 110 based on the extracted features received by the server 150 from the device 105. In some embodiments, the symptom assessment application 170 may determine an assessment of the one or more symptoms of Alzheimer's disease in the subject 110 based on the extracted features of the sensor data received from the device 105 and a patient database 175 stored in the memory 160. In some embodiments, the patient database 175 may include patient and/or clinical data. In some embodiments, the patient database 175 may include in-clinic and sensor-based measures of motor and cognitive function at baseline and longitudinal from mild Alzheimer's disease patients. In some embodiments, the patient database 175 may include data from patients at other stages of Alzheimer's disease, e.g. prodromal, moderate or severe. In some embodiments, the patient database 175 may be independent of the server 150. In some embodiments, the server 150 sends the determined assessment of the one or more symptoms of Alzheimer's disease in the subject 110 to the device 105. In some embodiments, the device 105 may output the assessment of the one or more symptoms of Alzheimer's disease. In some embodiments, the device 105 may communicate information to the subject 110 based on the assessment. In some embodiments, the assessment of the one or more symptoms of Alzheimer's disease may be communicated to a clinician that may determine individualized therapy for the subject 110 based on the assessment.

In some embodiments, the computer-instructions for the symptom monitoring application 130, when executed by the at least one processor 115, cause the device 105 to assess one or more symptoms of Alzheimer's disease in the subject 110 based on active testing of the subject 110. The device 105 prompts the subject 110 to perform one or more diagnostic tasks. In some embodiments, prompting the subject to perform the one or more diagnostic tasks includes prompting the subject to transcribe pre-specified sentences or prompting the subject to perform one or more actions. In some embodiments, the diagnostic tasks are anchored in or modelled after well-established methods and standardized tests for evaluating and assessing Alzheimer's disease.

In response to the subject 110 performing the one or more diagnostic tasks, the diagnostic device 105 receives a plurality of sensor data via the one or more sensors associated with the device 105. As mentioned above, the sensors associated with the device 105 may include a first sensor 120a that is disposed within the device 105 and a second sensor 120b that is worn by the subject 110. The device 105 receives a plurality of first sensor data via the first sensor 120a and a plurality of second sensor data via the second sensor 120b. In some embodiments, the one or more diagnostic tasks may be associated with at least one of a Fairytale test, 30 sec Walk Dual task, and a semantic memory test.

The device 105 extracts, from the received plurality of first sensor data and the received plurality of second sensor data, features associated with one or more symptoms of Alzheimer's disease in the subject 110. The symptoms of Alzheimer's disease in the subject 110 may include a symptom indicative of a cognitive function of the subject 110, a symptom indicative of a motor function of the subject 110, or a symptom indicative of a functional capacity of the subject 110. In some embodiments, the one or more symptoms of Alzheimer's disease in the subject 110 are indicative of at least one of visual attention, motor speed, cognitive processing speed, visuo-motor coordination or fine motor impairment. As discussed above, location-based data from a GPS or similar system may be used to assess symptoms related to the motor function and/or mobility of the subject and other location based assessments. Similarly, as discussed above, WiFi and Bluetooth signal density may be used, e.g., to help assess patent sociability.

The device 105 determines an assessment of the one or more symptoms of Alzheimer's disease in the subject 110 based on the extracted features of the received first and second sensor data. In some embodiments, the device 105 sends the extracted features over a network 180 to a server 150. The server 150 may include at least one processor 155 and a memory 161 storing computer-instructions for a symptom assessment application 170 that, when executed by the server processor 155, cause the processor 155 to determine an assessment of the one or more symptoms of Alzheimer's disease in the subject 110 based on the extracted features received by the server 150 from the device 105. In some embodiments, the symptom assessment application 170 may determine an assessment of the one or more symptoms of Alzheimer's disease in the subject 110 based on the extracted features of the sensor data received from the device 105 and a patient database 175 stored in the memory 160. In some embodiments, the patient database 175 may include patient and/or clinical data. In some embodiments, the patient database 175 may include in-clinic and sensor-based measures of motor and cognitive function at baseline and longitudinal from mild Alzheimer's disease patients. In some embodiments, the patient database 175 may include data from patients at other stages of Alzheimer's disease, e.g. like prodromal, moderate or severe. In some embodiments, the patient database 175 may be independent of the server 150. In some embodiments, the server 150 sends the determined assessment of the one or more symptoms of Alzheimer's disease in the subject 110 to the device 105. In some embodiments, the device 105 may output the assessment of the one or more symptoms of Alzheimer's disease. In some embodiments, the device 105 may communicate information to the subject 110 based on the assessment. In some embodiments, the assessment of the one or more symptoms of Alzheimer's disease may be communicated to a clinician that may determine individualized therapy for the subject 110 based on the assessment.

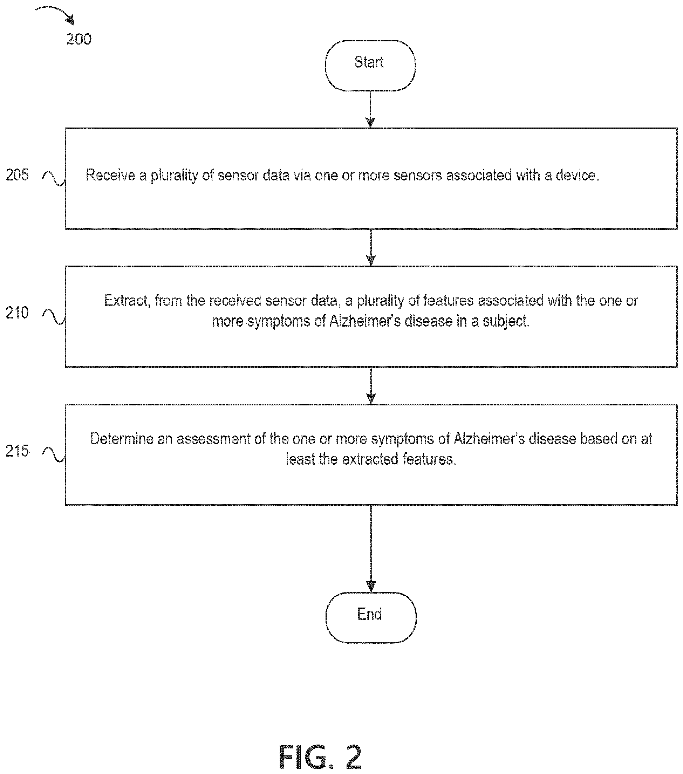

FIG. 2 illustrates an example method 200 for assessing one or more symptoms of Alzheimer's disease in a subject based on passive monitoring of the subject using the example device 105 of FIG. 1. While FIG. 2 is described with reference to FIG. 1, it should be noted that the method steps of FIG. 2 may be performed by other systems. The method 200 for assessing one or more symptoms of Alzheimer's disease in a subject includes receiving a plurality of sensor data via one or more sensors associated with a device (step 205). The method 200 includes extracting, from the received plurality of first sensor data, a plurality of features associated with the one or more symptoms of Alzheimer's disease in the subject (step 210). The method 200 also includes determining a first assessment of the one or more symptoms of Alzheimer's disease based on the extracted features (step 215).

The term "sensor data" as used herein refers to data different types of measurements, e.g. inter-key intervals and keystroke measures, word initiation effect, mean time and variability to type characters, amount and type of errors, lag time for first keystroke after errors and the like.

FIG. 2 sets forth an example method 200 for assessing one or more symptoms of Alzheimer's disease using the example device 105 in FIG. 1. In some embodiments, the device 105 may be a smartphone, a smartwatch or other mobile computing device. The device 105 includes at least one processor 115 and a memory 125 storing computer-instructions for a symptom monitoring application 130 that, when executed by the at least one processor 115, cause the device 105 to assess the one or more symptoms of Alzheimer's disease in the subject 110 based on passive monitoring of the subject 110.

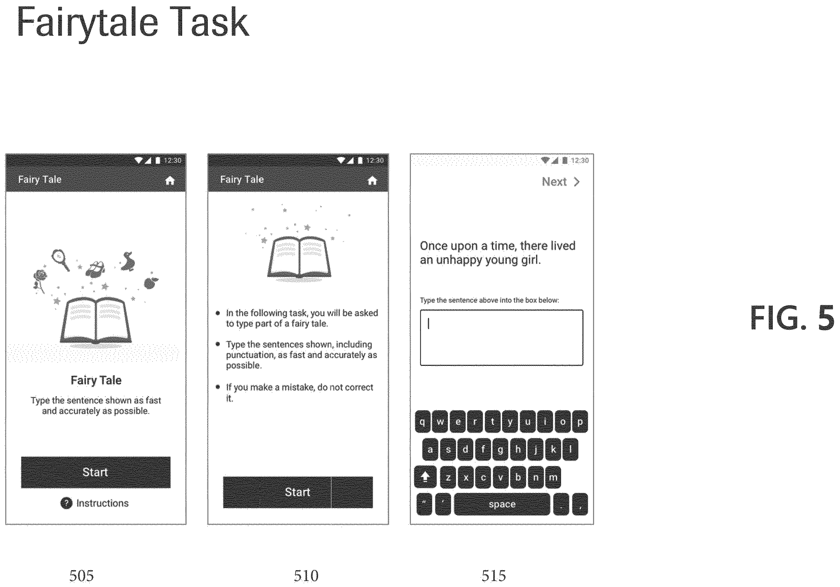

In some embodiments, the symptom monitoring application 130 may provide a diagnostic application that includes a user interface (UI) that is displayed on the display screen 160 of the device 105. In some embodiments, the display screen 160 may be a touchscreen and the user interacts with the diagnostic application via the displayed UI. FIGS. 5-7 depict example screenshots, illustrating the UI of an example diagnostic application according to illustrative aspects described herein, and responsive UI changes to the user interface as a user interacts with the diagnostic application.

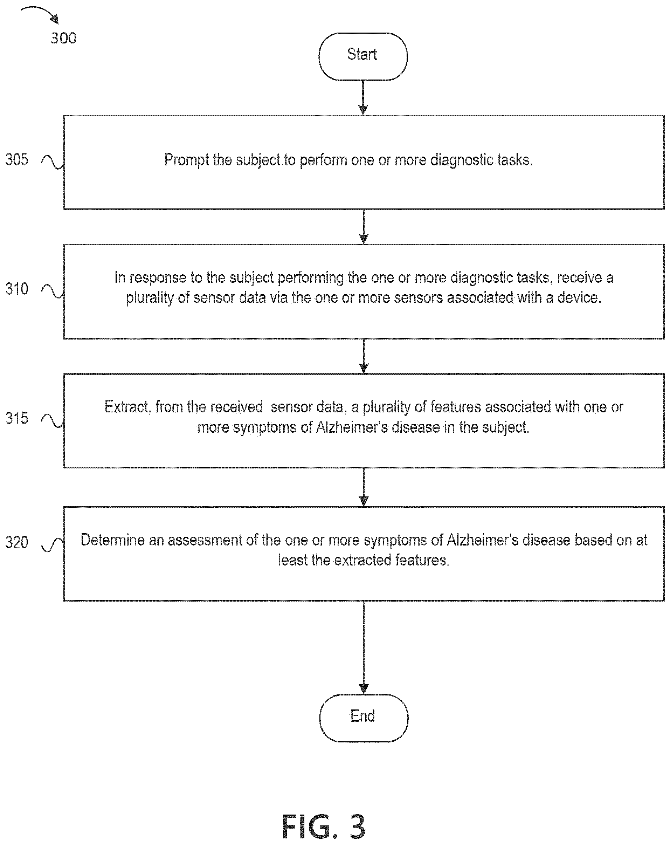

FIG. 3 illustrates an example method 300 for assessing one or more symptoms of Alzheimer's disease in a subject based on active testing of the subject using the example device 105 of FIG. 1. While FIG. 3 is described with reference to FIG. 1, it should be noted that the method steps of FIG. 3 may be performed by other systems. The method 300 includes prompting the subject to perform one or more diagnostic tasks (305). The method 300 includes receiving, in response to the subject performing the one or more tasks, a plurality of sensor data via the one or more sensors (step 310). The method 300 includes extracting, from the received sensor data, a plurality of features associated with one or more symptoms of Alzheimer's disease (315). The method 300 includes determining an assessment of the one or more symptoms of Alzheimer's disease based on at least the extracted sensor data (step 320).

FIG. 3 sets forth an example method 300 for assessing one or more symptoms of Alzheimer's disease based on active testing of the subject 110 using the example device 105 in FIG. 1. In some embodiments, active testing of the subject 110 using the device 105 may be selected via the user interface of the symptom monitoring application 130.

The method 300 begins by proceeding to step 305 which includes prompting the subject to perform one or more diagnostic tasks. The device 105 prompts the subject 110 to perform one or more diagnostic tasks. In some embodiments, prompting the subject to perform the one or more diagnostic tasks includes prompting the subject to answer one or more questions or prompting the subject to perform one or more actions. In some embodiments, the diagnostic tasks are anchored in or modelled after well-established methods and standardized tests for evaluating and assessing Alzheimer's disease.

In some embodiments, the diagnostic tasks may include to transcribe pre-specified sentences to assess semantic memory of the patient at the time of the active testing. The patient's response to task provide an assessment of the patient's daily disease fluctuations and may be used as a control when assessing symptoms associated with motor and cognitive functions of the patient.

In some embodiments, the diagnostic tasks may include a Fairytale test, 30 sec Walk Dual task, and a semantic memory test.

The term "Fairytale test" as used herein describe a test where a subject is asked to transcribe pre-specified sentences on the device, in particular on the smartphone. Sentences are selected from a culturally adaptable story. The story is continued across the assessments. The subject is asked to read the entire sentence first and then start typing the sentence. To standardize the keyboard, functionalities like swipey keyboard, auto-correct, landscape keyboard mode and the delete key are disabled. As keyboard typing is a complex cognitive, perceptual and motor process, the task assesses semantic memory, processing speed, lexical knowledge, psychomotor slowing through inter-key intervals and keystroke measures in general, word initiation effect, mean time and variability to type characters, amount and type of errors, lag time for first keystroke after errors. If sentences are removed before the typing part, the task may also serve as episodic and semantic memory test.

Reference median time between each consecutive keystroke for healthy volunteers (HC) are values < 0.5 seconds.

The term "30 sec Walk Dual task" as used herein describe a test where a subject is asked to carry the device, in particular a smartphone, in a running belt or their pockets while walking for 30 seconds or approximately 50 meters (54 yards). As a dual task, the subject is asked to count down out loud in steps of 5s from a random, even number. Gait is monitored using accelerometers, gyroscope, and magnetometer in the device, in particular the smartphone. The countdown dual task is monitored using the device microphone. To help ensure correct task completion, the subject is asked to enter the number the subject counted down to at the end of the task. Divided attention impairs walking and balance abilities and is even more marked in elderly populations and often associated with falls. This task assesses different aspects of gait speed and variability, movement regularity, unsteadiness, sway path, number of times stopped walking, cadence, stance time, step detection, and attentional gait-related measures.

The term "semantic memory test" as used herein is an object features task based on knowledge of concepts. It is a test where the subject is asked to select or differentiate presented images, including images of words, with increasing difficulty level. The test further forces the subject to think about specific features of the concept that vary in distinctiveness, complexity, frequency, and feature types. The features are presented individually to the subject in order to avoid attention effects.

The method 300 proceeds to step 310 which includes in response to the subject performing the one or more diagnostics tasks, receiving, a plurality of second sensor data via the one or more sensors. In response to the subject 110 performing the one or more diagnostic tasks, the diagnostic device 105 receives, a plurality of sensor data via the one or more sensors associated with the device 105. As mentioned above, the sensors associated with the device 105 include a first sensor 120a that is disposed within the device 105 and a second sensor 120b that is worn by the subject 110. The device 105 receives a plurality of first sensor data via the first sensor 120a and a plurality of second sensor data via the second sensor 120b.

The method 300 proceeds to step 315 including extracting, from the received sensor data, a second plurality of features associated with one or more symptoms of Alzheimer's disease. The device 105 extracts, from the received first sensor data and second sensor data, features associated with one or more symptoms of Alzheimer's disease in the subject 110. The symptoms of Alzheimer's disease in the subject 110 may include a symptom indicative of a cognitive function of the subject 110, a symptom indicative of a motor function of the subject 110, a symptom indicative of a behavioral function of the subject 110, or a symptom indicative of a functional capacity of the subject 110. In some embodiments, the extracted features of the plurality of first and second sensor data may be indicative of symptoms of Alzheimer's disease such as visual attention, motor speed, cognitive processing speed, visuo-motor coordination or fine motor impairment. As discussed above, location-based data from a GPS or similar system may be used to assess symptoms related to the motor function and/or mobility of the subject and other location based assessments. Similarly, WiFi and Bluetooth signal density may be used to help assess patent sociability and the like.

The method 300 proceeds to step 320 which includes determining an assessment of the one or more symptoms of Alzheimer's disease based on at least the extracted sensor data. The device 105 determines an assessment of the one or more symptoms of Alzheimer's disease in the subject 110 based on the extracted features of the received first and second sensor data. In some embodiments, the device 105 may send the extracted features over a network 180 to a server 150. The server 150 includes at least one processor 155 and a memory 160 storing computer-instructions for a symptom assessment application 170 that, when executed by the processor 155, determine an assessment of the one or more symptoms of Alzheimer's disease in the subject 110 based on the extracted features received by the server 150 from the device 105. In some embodiments, the symptom assessment application 170 may determine an assessment of the one or more symptoms of Alzheimer's disease in the subject 110 based on the extracted features of sensor data received from the device 105 and a patient database 175 stored in the memory 160. The patient database 175 may include various clinical data. In some embodiments, the second device may be one or more wearable sensors. In some embodiments, the second device may be any device that includes a motion sensor with an inertial measurement unit (IMU). In some embodiments, the second device may be several devices or sensors. In some embodiments, the patient database 175 may be independent of the server 150. In some embodiments, the server 150 sends the determined assessment of the one or more symptoms of Alzheimer's disease in the subject 110 to the device 105. In some embodiments, such as in FIG. 1, the device 105 may output an assessment of the one or more symptoms of Alzheimer's disease on the display 160 of the device 105. In some embodiments, the assessment of the one or more symptoms of Alzheimer's disease may be communicated to a clinician that may determine individualized therapy for the subject 110 based on the assessment.

As discussed above, assessments of symptom severity and progression of Alzheimer's disease using diagnostics according to the present disclosure correlate sufficiently with the assessments based on clinical results and have the potential to replace clinical patient monitoring and testing. Diagnostics according to the present disclosure were studied in a group of Alzheimer's disease patients. The patients can be provided with a smartphone application that includes 3 active tests, or get otherwise access. The active tests included Fairytale test, 30 sec Walk Dual task, and a semantic memory test.

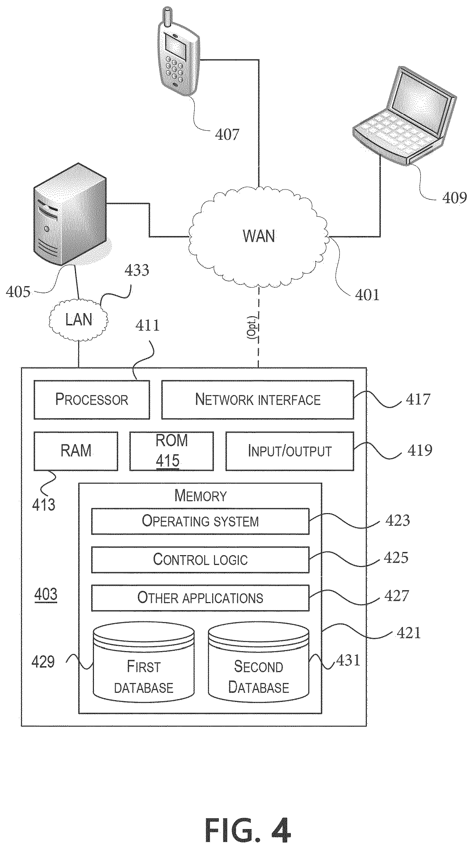

FIG. 4 illustrates one example of a network architecture and data processing device that may be used to implement one or more illustrative aspects described herein, such as the aspects described in FIGS. 1, 2 and 3. Various network nodes 403, 405, 407, and 409 may be interconnected via a wide area network (WAN) 401, such as the Internet. Other networks may also or alternatively be used, including private intranets, corporate networks, LANs, wireless networks, personal networks (PAN), and the like. Network 401 is for illustration purposes and may be replaced with fewer or additional computer networks. A local area network (LAN) may have one or more of any known LAN topology and may use one or more of a variety of different protocols, such as Ethernet. Devices 403, 405, 407, 409 and other devices (not shown) may be connected to one or more of the networks via twisted pair wires, coaxial cable, fiber optics, radio waves or other communication media.

The term "network" as used herein and depicted in the drawings refers not only to systems in which remote storage devices are coupled together via one or more communication paths, but also to stand-alone devices that may be coupled, from time to time, to such systems that have storage capability. Consequently, the term "network" includes not only a "physical network" but also a "content network," which is comprised of the data-attributable to a single entity-which resides across all physical networks.

The components may include data server 403, web server 405, and client computers 407, 409. Data server 403 provides overall access, control and administration of databases and control software for performing one or more illustrative aspects described herein. Data server 403 may be connected to web server 405 through which users interact with and obtain data as requested. Alternatively, data server 403 may act as a web server itself and be directly connected to the Internet. Data server 403 may be connected to web server 405 through the network 401 (e.g., the Internet), via direct or indirect connection, or via some other network. Users may interact with the data server 403 using remote computers 407, 409, e.g., using a web browser to connect to the data server 403 via one or more externally exposed web sites hosted by web server 405. Client computers 407, 409 may be used in concert with data server 403 to access data stored therein, or may be used for other purposes. For example, from client device 407 a user may access web server 405 using an Internet browser, as is known in the art, or by executing a software application that communicates with web server 405 and/or data server 403 over a computer network (such as the Internet). In some embodiments, the client computer 407 may be a smartphone, smartwatch or other mobile computing device, and may implement a diagnostic device, such as the device 105 shown in FIG. 1. In some embodiments, the data server 403 may implement a server, such as the server 150 shown in FIG. 1.

Servers and applications may be combined on the same physical machines, and retain separate virtual or logical addresses, or may reside on separate physical machines. FIG. 1 illustrates just one example of a network architecture that may be used, and those of skill in the art will appreciate that the specific network architecture and data processing devices used may vary, and are secondary to the functionality that they provide, as further described herein. For example, services provided by web server 405 and data server 403 may be combined on a single server.

Each component 403, 405, 407, 409 may be any type of known computer, server, or data processing device. Data server 403, e.g., may include a processor 411 controlling overall operation of the rate server 403. Data server 403 may further include RAM 413, ROM 415, network interface 417, input/output interfaces 419 (e.g., keyboard, mouse, display, printer, etc.), and memory 421. I/O 419 may include a variety of interface units and drives for reading, writing, displaying, and/or printing data or files. Memory 421 may further store operating system software 423 for controlling overall operation of the data processing device 403, control logic 425 for instructing data server 403 to perform aspects described herein, and other application software 427 providing secondary, support, and/or other functionality which may or may not be used in conjunction with other aspects described herein. The control logic may also be referred to herein as the data server software 425. Functionality of the data server software may refer to operations or decisions made automatically based on rules coded into the control logic, made manually by a user providing input into the system, and/or a combination of automatic processing based on user input (e.g., queries, data updates, etc.).

Memory 421 may also store data used in performance of one or more aspects described herein, including a first database 429 and a second database 431. In some embodiments, the first database may include the second database (e.g., as a separate table, report, etc.). That is, the information can be stored in a single database, or separated into different logical, virtual, or physical databases, depending on system design. Devices 405, 407, 409 may have similar or different architecture as described with respect to device 403. Those of skill in the art will appreciate that the functionality of data processing device 403 (or device 405, 407, 409) as described herein may be spread across multiple data processing devices, for example, to distribute processing load across multiple computers, to segregate transactions based on geographic location, user access level, quality of service (QoS), etc.

One or more aspects described herein may be embodied in computer-usable or readable data and/or computer-executable instructions, such as in one or more program modules, executed by one or more computers or other devices as described herein. Generally, program modules include routines, programs, objects, components, data structures, etc. that perform particular tasks or implement particular abstract data types when executed by a processor in a computer or other device. The modules may be written in a source code programming language that is subsequently compiled for execution, or may be written in a scripting language such as (but not limited to) HTML or XML. The computer executable instructions may be stored on a computer readable medium such as a hard disk, optical disk, removable storage media, solid state memory, RAM, etc. As will be appreciated by one of skill in the art, the functionality of the program modules may be combined or distributed as desired in various embodiments. In addition, the functionality may be embodied in whole or in part in firmware or hardware equivalents such as integrated circuits, field programmable gate arrays (FPGA), and the like. Particular data structures may be used to more effectively implement one or more aspects, and such data structures are contemplated within the scope of computer executable instructions and computer-usable data described herein.

FIG. 5 depict example screenshots 505, 510 and 515 illustrating an example diagnostic application according to one or more illustrative aspects described herein. The screenshot 505 of FIG. 5 shows the introduction screen of the Fairytale test. Screenshot 510 showing the second screen with detailed instructions of how to perform the task. The user needs to select "Start" to begin with the fairy tale task. Screenshot 515 illustrating a selection of an example sentence that the subject should read out and type into a box.

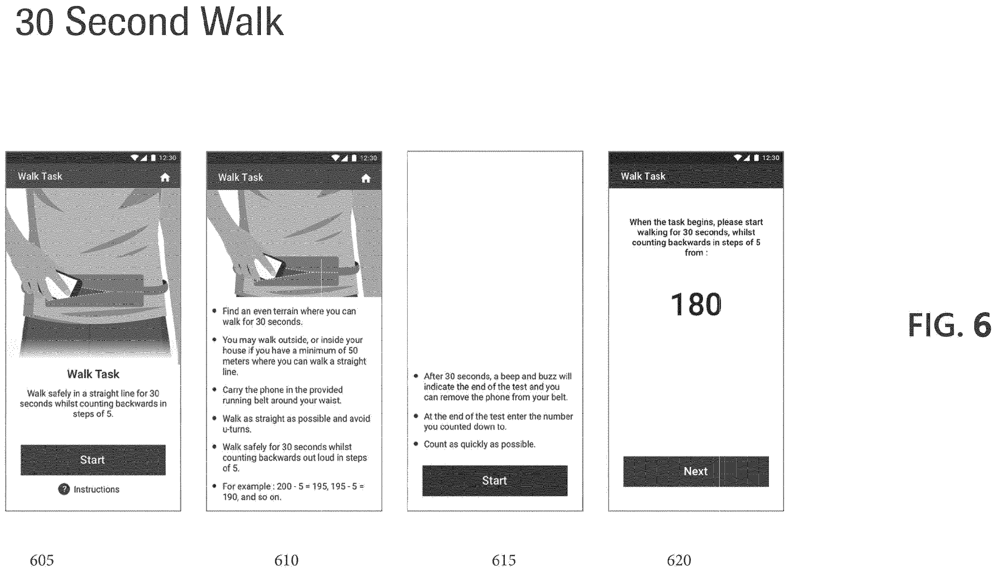

FIG. 6 depict example screenshots 605, 610, 615, and 620 illustrating an example diagnostic application according to one or more illustrative aspects described herein. The screenshot 605 of FIG. 5 shows the introduction screen of the 30 sec Walk Dual task. Screenshots 610 and 615 showing the second third screen with detailed instructions of how to perform the task. Screenshot 515 illustrating a selection of an example number that the subject is asked to count down.

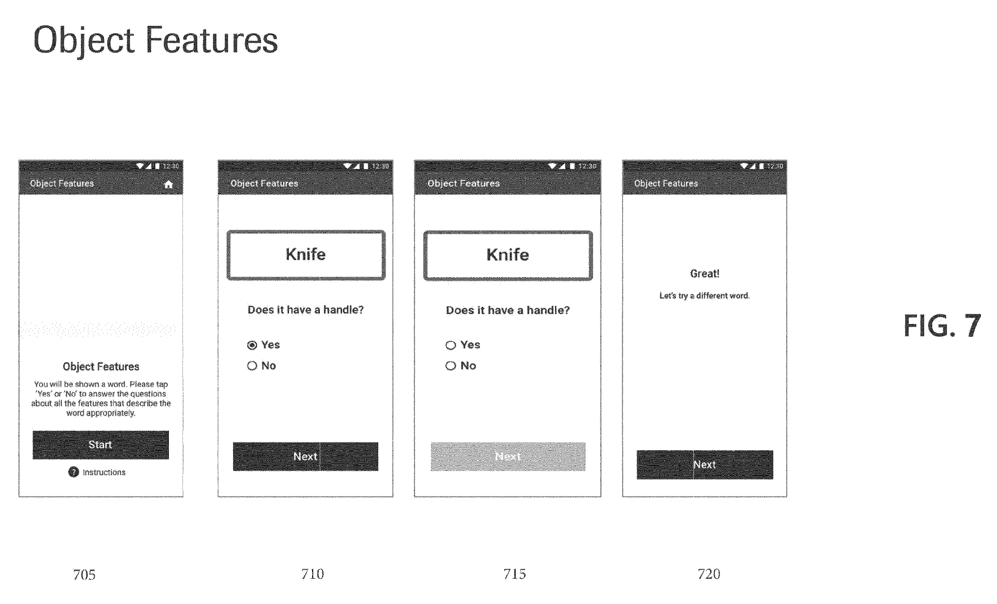

FIG. 7 depict example screenshots 705, 710, 715, and 720 illustrating an example diagnostic application according to one or more illustrative aspects described herein. The screenshot 705 of FIG. 5 shows the introduction screen of the semantic memory test. Screenshots 710 and 715 showing each a selection of an example term in the box along with a question to a specific feature of that term in the box. Screenshot 720 gives an example of an intermediate screen that announces to the patient that the next part of the test is about to start.

Although the subject matter has been described in language specific to structural features and/or methodological acts, it is to be understood that the subject matter defined in the appended claims is not necessarily limited to the specific features or acts described above. Rather, the specific features and acts described above are disclosed as illustrative forms of implementing the claims.

### EXAMPLE

EX. 1 gives results of a Fairytale test in 7 subjects.
a) The time between 2 keystrokes in these subjects performing a Fairytale test has been measured at selected parts of the texts and mean values have been determined.
Median time between keystrokes differs across individuals: 0.47s (min 0.32s - max 0.96s)
The time between successive keystrokes decreases as the subject progresses through the text:
   Median time between keystrokes (first half of text): 0.48s (min 0.32s - max 1.05s)
   Median time between keystrokes (second half of text): 0.45s (min 0.30s - max 0.91s)

b) Further attention was paid to the time difference of selected characters.
Special characters produce a longer time between keystrokes:
- all characters: median 0.45s (min 0.09 - max 21.29s)
- dot (" . "): median 1.78s (min 0.228s - max 21.30s)
- comma (" , "): median 1.47s (min 0.5s - max 1.47s)
- single quote (" ' "): median 1.94s (min 1.75s - max 2.12s)

| Subject | | Category | | S-MoCA | | Gender | | Age | | Keystroke count | | Median time between each consecutive keystroke (s) | |
|---|---|---|---|---|---|---|---|---|---|---|---|---|---|
| | p13 | | HC | | 13 | | M | | 64 | | 171 | | 0.34 |
| | p14 | | HC | | 16 | | F | | 69 | | 67 | | 0.465 |
| | p15 | | HC | | 14 | | M | | 77 | | 125 | | 0.479 |
| | p11 | | SCC | | 13 | | F | | 64 | | 120 | | 0.316 |
| | p10 | | SCC | | 13 | | M | | 71 | | 199 | | 0.474 |
| | p03 | SCC | | | 13 | | F | | 59 | | 54 | | 0.8205 |
| | p02 | Mild AD | | | 10 | | F | | 75 | | 44 | | 0.958 |

The terms have the following meaning throughout the specification: HC = Healthy Control; SCC = Subjective Cognitive Complaint; Mild AD = mild Alzheimer's disease; S-MoCa (see https://www.alz.org/careplanning/downloads/short-moca.pdf)

## Claims

1. A diagnostic device for assessing one or more pre-clinical signs and/or symptoms of Alzheimer's disease in a subject, the device comprising:
at least one processor;
one or more sensors associated with the device; and
memory storing computer-readable instructions that, when executed by the at least one processor, cause the device to:
receive a plurality of first sensor data via the one or more sensors associated with the device;
extract, from the received first sensor data, a first plurality of features associated with the one or more symptoms of Alzheimer's disease in the subject; and
determine a first assessment of the one or more symptoms of Alzheimer's disease based on the extracted first plurality of features.
prompt the subject to perform one or more diagnostic tasks;
in response to the subject performing the one or more diagnostic tasks, receive a plurality of second sensor data via the one or more sensors associated with the device;
extract, from the received second sensor data, a second plurality of features associated with the one or more symptoms of Alzheimer's disease; and
determine a second assessment of the one or more symptoms of Alzheimer's disease based on the extracted second plurality of features,
**characterised in that**
prompting the subject to perform the one or more diagnostic tasks includes prompting the subject to transcribe one or more pre-specified sentences comprising special characters.

2. The device of any one of claim 1, wherein the one or more symptoms of Alzheimer's disease in the subject include at least one of a symptom indicative of a cognitive function of the subject, a symptom indicative of a motor function of the subject, or a symptom indicative of a functional capacity of the subject.

3. The device of any one of claims 1-2, wherein the device is a smartphone or smartwatch, in particular a smartphone.

4. The device of any one of claims 1-3, wherein the one or more diagnostic tasks are associated with at least one of a Fairytale test, and a semantic memory test.

5. A computer-implemented method for assessing one or more symptoms of Alzheimer's disease in a subject, the method comprising:
receiving a plurality of first sensor data via one or more sensors associated with a device;
extracting, from the received first sensor data, a first plurality of features associated with the one or more symptoms of Alzheimer's disease in the subject; and
determining a first assessment of the one or more symptoms of Alzheimer's disease based on the extracted first plurality of features,
prompting the subject to perform one or more diagnostic tasks;
in response to the subject performing the one or more diagnostics tasks, receiving, a plurality of second sensor data via the one or more sensors;
extracting, from the received second sensor data, a second plurality of features associated with one or more symptoms of Alzheimer's disease; and
determining a second assessment of the one or more symptoms of Alzheimer's disease based on at least the extracted second sensor data,
**characterised in that**
prompting the subject to perform the one or more diagnostic tasks includes prompting the subject to transcribe one or more pre-specified sentences comprising special characters.

6. The computer-implemented method of of claim 5, wherein the one or more symptoms of Alzheimer's disease in the subject include at least one of a symptom indicative of a cognitive function of the subject, a symptom indicative of a motor function of the subject, or a symptom indicative of a functional capacity of the subject, in particular wherein the one or more symptoms of Alzheimer's disease in the subject are indicative of at least one of visual attention, motor speed, cognitive processing speed, visuo-motor coordination or fine motor impairment.

7. The computer-implemented method of any one of claims 5-6, whereby the subject's mobility is assessed at least partly based on accelerometers, gyroscope, and/or magnetometer data, whereby the subject's cognitive function is assessed at least partly based on inter-key intervals and keystroke measures in general, word initiation effect, mean time and variability to type characters, amount and type of errors and/or lag time for first keystroke after errors, and whereby the subject's functional capacity is assessed at least partly based on a semantic task.

8. The computer-implemented method of any one of claims 5-7, wherein the one or more diagnostic tasks are associated with at least one of a Fairytale test, and a semantic memory test.

9. A method of identifying a patient subpopulation based on a computer-implemented method of any one of claims 5-8.

## Patentansprüche

1. Diagnosevorrichtung zum Bewerten von einem oder mehreren vorklinischen Anzeichen und/oder Symptom(en) von Alzheimer-Krankheit bei einem Individuum, wobei die Vorrichtung Folgendes umfasst:
mindestens einen Prozessor;
einen oder mehrere Sensor(en), der/die mit der Vorrichtung in Verbindung steht/stehen, und
einen Speicher, der computerlesbare Anweisungen speichert, die beim Ausführen von dem mindestens einen Prozessor die Vorrichtung zu Folgendem veranlassen:
Empfangen einer Vielzahl von ersten Sensordaten über den einen oder die mehreren Sensor(en), der/die mit der Vorrichtung in Verbindung steht/stehen;
Extrahieren einer ersten Vielzahl von Merkmalen, die mit dem einen oder den mehreren Symptom(en) von Alzheimer-Krankheit bei dem Individuum in Verbindung stehen, aus den empfangenen ersten Sensordaten und
Bestimmen einer ersten Bewertung des einen Symptoms oder der mehreren Symptome von Alzheimer-Krankheit basierend auf der extrahierten ersten Vielzahl von Merkmalen;
Auffordern des Individuums, eine oder mehrere Diagnoseaufgabe(n) auszuführen;
als Reaktion darauf, dass das Individuum die eine oder mehreren Diagnoseaufgabe(n) ausführt, Empfangen einer Vielzahl von zweiten Sensordaten über den einen oder die mehreren Sensor(en), der/die mit der Vorrichtung in Verbindung steht/stehen;
Extrahieren einer zweiten Vielzahl von Merkmalen, die mit dem einen oder den mehreren Symptom(en) von Alzheimer-Krankheit in Verbindung stehen, aus den empfangenen zweiten Sensordaten und
Bestimmen einer zweiten Bewertung des einen Symptoms oder der mehreren Symptome von Alzheimer-Krankheit basierend auf der extrahierten zweiten Vielzahl von Merkmalen,
**dadurch gekennzeichnet, dass** das Auffordern des Individuums, die eine oder mehreren Diagnoseaufgabe(en) auszuführen, das Auffordern des Individuums, einen vorspezifizierten Satz oder mehrere vorspezifizierte Sätze umzuschreiben, der/die Sonderzeichen umfasst/umfassen, einschließt.

2. Vorrichtung nach Anspruch 1, wobei das eine oder die mehreren Symptom(e) von Alzheimer-Krankheit bei dem Individuum mindestens eines von einem Symptom, das eine kognitive Funktion des Individuums angibt, einem Symptom, das eine motorische Funktion des Individuums angibt, oder einem Symptom, das eine Funktionsfähigkeit des Individuums angibt, einschließt/einschließen.

3. Vorrichtung nach einem der Ansprüche 1-2, wobei die Vorrichtung ein Smartphone oder eine Smartwatch, insbesondere ein Smartphone ist.

4. Vorrichtung nach einem der Ansprüche 1-3, wobei die eine oder mehreren Diagnoseaufgabe(n) mit mindestens einem von einem Märchentest und einem semantischen Gedächtnistest verbunden ist/sind.

5. Computerimplementiertes Verfahren zum Bewerten eines Symptoms oder mehrerer Symptome von Alzheimer-Krankheit bei einem Individuum, wobei das Verfahren Folgendes umfasst:
Empfangen einer Vielzahl von ersten Sensordaten über einen oder mehrere Sensor(en), der/die mit einer Vorrichtung in Verbindung steht/stehen;
Extrahieren einer ersten Vielzahl von Merkmalen, die mit dem einen oder den mehreren Symptom(en) von Alzheimer-Krankheit bei dem Individuum in Verbindung stehen, aus den empfangenen ersten Sensordaten und
Bestimmen einer ersten Bewertung des einen Symptoms oder der mehreren Symptome von Alzheimer-Krankheit basierend auf der extrahierten ersten Vielzahl von Merkmalen;
Auffordern des Individuums, eine oder mehrere Diagnoseaufgabe(n) auszuführen;
als Reaktion darauf, dass das Individuum die eine oder mehreren Diagnoseaufgabe(n) ausführt, Empfangen einer Vielzahl von zweiten Sensordaten über den einen oder die mehreren Sensor(en);
Extrahieren einer zweiten Vielzahl von Merkmalen, die mit dem einen oder den mehreren Symptom(en) von Alzheimer-Krankheit in Verbindung stehen, aus den empfangenen zweiten Sensordaten und
Bestimmen einer zweiten Bewertung des einen Symptoms oder der mehreren Symptome von Alzheimer-Krankheit basierend auf zumindest den extrahierten zweiten Sensordaten,
**dadurch gekennzeichnet, dass** das Auffordern des Individuums, die eine oder mehreren Diagnoseaufgabe(en) auszuführen, das Auffordern des Individuums, einen vorspezifizierten Satz oder mehrere vorspezifizierte Sätze umzuschreiben, der/die Sonderzeichen umfasst/umfassen, einschließt.

6. Computerimplementiertes Verfahren nach Anspruch 5, wobei das eine oder die mehreren Symptom(e) von Alzheimer-Krankheit bei dem Individuum mindestens eines von einem Symptom, das eine kognitive Funktion des Individuums angibt, einem Symptom, das eine motorische Funktion des Individuums angibt, oder einem Symptom, das eine Funktionsfähigkeit des Individuums angibt, einschließt/einschließen, insbesondere wobei das eine oder die mehreren Symptom(e) von Alzheimer-Krankheit bei dem Individuum mindestens eines von visueller Aufmerksamkeit, motorischer Geschwindigkeit, kognitiver Verarbeitungsgeschwindigkeit, visuomotorischer Koordination oder feinmotorischer Beeinträchtigung angibt/angeben.

7. Computerimplementiertes Verfahren nach einem der Ansprüche 5-6, wobei die Mobilität des Individuums mindestens teilweise basierend auf Beschleunigungsmesser-, Gyroskop- und/oder Magnetometerdaten bewertet wird, wobei die kognitive Funktion des Individuums mindestens teilweise basierend auf Zwischentastintervallen und Anschlagsmessungen im Allgemeinen, Wortinitiierungseffekt, mittlerer Zeit und Variabilität zum Tippen von Zeichen, Anzahl und Art von Fehlern und/oder Verzögerungszeit für einen ersten Anschlag nach Fehlern bewertet wird und wobei die Funktionsfähigkeit des Individuums mindestens teilweise basierend auf einer semantischen Aufgabe bewertet wird.

8. Computerimplementiertes Verfahren nach einem der Ansprüche 5-7, wobei die eine oder mehreren Diagnoseaufgabe(n) mit mindestens einem von einem Märchentest und einem semantischen Gedächtnistest verbunden ist/sind.

9. Verfahren zum Identifizieren einer Patientensubpopulation basierend auf einem computerimplementierten Verfahren nach einem der Ansprüche 5-8.

## Revendications

1. Dispositif de diagnostic pour évaluer un ou plusieurs signes et/ou symptômes précliniques de la maladie d'Alzheimer chez un sujet, le dispositif comprenant :
au moins un processeur ;
un ou plusieurs capteurs associés au dispositif ; et
une mémoire stockant des instructions lisibles par ordinateur qui, lorsqu'elles sont exécutées par l'au moins un processeur, amènent le dispositif à :
recevoir une pluralité de premières données de capteurs par l'intermédiaire du ou des capteurs associés au dispositif ;
extraire, à partir des premières données de capteurs reçues, une première pluralité de caractéristiques associées au ou aux symptômes de la maladie d'Alzheimer chez le sujet ; et
déterminer une première évaluation du ou des symptômes de la maladie d'Alzheimer sur la base de la première pluralité de caractéristiques extraites,
inviter le sujet à effectuer une ou plusieurs tâches de diagnostic ;
en réponse à la réalisation par le sujet de la ou des tâches de diagnostic, recevoir une pluralité de secondes données de capteurs par l'intermédiaire du ou des capteurs associés au dispositif ;
extraire, à partir des secondes données de capteurs reçues, une seconde pluralité de caractéristiques associées au ou aux symptômes de la maladie d'Alzheimer ; et
déterminer une seconde évaluation du ou des symptômes de la maladie d'Alzheimer sur la base de la seconde pluralité de caractéristiques extraites,
**caractérisé en ce que** l'invite du sujet à réaliser la ou les tâches de diagnostic comprend l'invite du sujet à transcrire une ou plusieurs phrases pré-spécifiées comprenant des caractères spéciaux.

2. Dispositif selon l'une quelconque de la revendication 1, dans lequel le ou les symptômes de la maladie d'Alzheimer chez le sujet comprennent au moins un parmi un symptôme indiquant une fonction cognitive du sujet, un symptôme indiquant une fonction motrice du sujet ou un symptôme indiquant une capacité fonctionnelle du sujet.

3. Dispositif selon l'une quelconque des revendications 1 à 2, dans lequel le dispositif est un smartphone ou une montre connectée, en particulier un smartphone.

4. Dispositif selon l'une quelconque des revendications 1 à 3, dans lequel la ou les tâches de diagnostic sont associées à au moins un parmi un test de Fairytale et un test de mémoire sémantique.

5. Procédé mis en œuvre par ordinateur pour l'évaluation d'un ou de plusieurs symptômes de la maladie d'Alzheimer chez un sujet, le procédé comprenant :
la réception d'une pluralité de premières données de capteurs par l'intermédiaire d'un ou de plusieurs capteurs associés à un dispositif ;
l'extraction, à partir des premières données de capteurs reçues, d'une première pluralité de caractéristiques associées au ou aux symptômes de la maladie d'Alzheimer chez le sujet ; et
la détermination d'une première évaluation du ou des symptômes de la maladie d'Alzheimer sur la base de la première pluralité de caractéristiques extraites,
l'invite du sujet à effectuer une ou plusieurs tâches de diagnostic ;
en réponse à la réalisation par le sujet de la ou des tâches de diagnostic, la réception d'une pluralité de secondes données de capteurs par l'intermédiaire du ou des capteurs ;
l'extraction, à partir des secondes données de capteurs reçues, d'une seconde pluralité de caractéristiques associées à un ou plusieurs symptômes de la maladie d'Alzheimer ; et
la détermination d'une seconde évaluation du ou des symptômes de la maladie d'Alzheimer sur la base d'au moins les secondes données de capteurs extraites,
**caractérisé en ce que** l'invite du sujet à réaliser la ou les tâches de diagnostic comprend l'invite du sujet à transcrire une ou plusieurs phrases pré-spécifiées comprenant des caractères spéciaux.

6. Procédé mis en œuvre par ordinateur selon la revendication 5, dans lequel le ou les symptômes de la maladie d'Alzheimer chez le sujet comprennent au moins un parmi un symptôme indiquant une fonction cognitive du sujet, un symptôme indiquant une fonction motrice du sujet ou un symptôme indiquant une capacité fonctionnelle du sujet, en particulier dans lequel le ou les symptômes de la maladie d'Alzheimer chez le sujet indiquent au moins un parmi l'attention visuelle, la vitesse motrice, la vitesse de traitement cognitif, la coordination visuomotrice ou la déficience motrice fine.

7. Procédé mis en œuvre par ordinateur selon l'une quelconque des revendications 5 à 6, moyennant quoi la mobilité du sujet est évaluée au moins en partie en se basant sur des données d'accéléromètres, de gyroscope et/ou de magnétomètre, moyennant quoi la fonction cognitive du sujet est évaluée au moins en partie en se basant sur des mesures d'intervalles intertouche et de frappe de touche en général, un effet d'initiation de mots, le temps moyen et la variabilité pour saisir des caractères, la quantité et le type d'erreurs et/ou le temps de latence pour la première frappe de touche après des erreurs, et moyennant quoi la capacité fonctionnelle du sujet est évaluée au moins en partie en se basant sur une tâche sémantique.

8. Procédé mis en œuvre par ordinateur selon l'une quelconque des revendications 5 à 7, dans lequel la ou les tâches de diagnostic sont associées à au moins un parmi un test de Fairytale et un test de mémoire sémantique.

9. Procédé d'identification d'une sous-population de patients en se basant sur un procédé mis en œuvre par ordinateur selon l'une quelconque des revendications 5 à 8.
